# EUROPEAN PATENT SPECIFICATION

(11) **EP 3 986 247 B1**
(45) Date of publication and mention of the grant of the patent: **06.03.2024**
(21) Application number: 19929080.0
(22) Date of filing: 15.05.2019
(51) Int. Cl.: A61B 5/00, A61B 5/103, A61N 5/06, F21L 4/02, F21V 23/04

(54) **PORTABLE BREAST LIGHT ASSEMBLY**
TRAGBARE BRUSTLEUCHTENANORDNUNG
ENSEMBLE LUMIÈRE MAMMAIRE PORTABLE

(43) Date of publication of application: 27.04.2022
(73) Proprietor: Power Productions Group LLC, Miami, FL 33127 (US)
(72) Inventor: YANG, Lin, 41462 Neuss (DE)
(74) Representative: Capitán García, Maria Nuria
(86) International application number: PCT/US2019/032430
(87) International publication number: WO 2020/231422

(56) References cited:
- CN-A- 106 419 842
- US-A1- 2011 190 638
- KWOK: "Silkpro PINK luminous breast", , 9 May 2017 (2017-05-09), XP054981248, Retrieved from the Internet: URL:https://www.youtube.com/watch?v=_i4FQs a7Q6I&feature=youtu.be

## Description

### II. FIELD OF THE INVENTION

The present invention relates to light assemblies, and more particularly, to portable light assemblies to examine breasts.

### III. DESCRIPTION OF THE RELATED ART

Applicant believes that one of the closest references corresponds to U.S. Patent No. 4,286,602 issued to Robert Guy on September 1, 1981 for Transillumination diagnostic system.

Applicant believes that another reference corresponds to U.S. Patent Application Publication No. 5,683,350 published on November 4, 1997 to Paul et al. for Oral transluminating device.

Applicant believes that another reference corresponds to U.S. Patent No. 5,799,656 issued to Alfano, et al. on September 1, 1998 for Optical imaging of breast tissues to enable the detection therein of calcification regions suggestive of cancer.

Applicant believes that another reference corresponds to U.S. Patent No. 6,148,223 issued to Davis, et al. on November 14, 2000 for Transilluminator device.

Applicant believes that another reference corresponds to U.S. Patent No. 6,230,046 issued to Crane, et al. on May 8, 2001 for Optical imaging of breast tissues to enable the detection therein of calcification regions suggestive of cancer.

Applicant believes that another reference corresponds to U.S. Patent No. 7,155,273 issued to Geoffrey L. Taylor on December 26, 2006 for Blanching response pressure sore detector apparatus and method.

Applicant believes that another reference corresponds to U.S. Patent No. 7,431,695 issued to Frank Creaghan on October 7, 2008 for Neonatal transilluminator apparatus.

Applicant believes that another reference corresponds to U.S. Patent No. 8,032,205 issued to Nizar A. Mullani on October 4, 2011 for Transilluminator light shield.

Applicant believes that another reference corresponds to U.S. Patent No. 8,231,542 issued to Keith, et al. on July 31, 2012 for System for analyzing thermal data based on breast surface temperature to determine suspect conditions.

Applicant believes that another reference corresponds to U.S. Patent No. 8,388,523 issued to Vivenzio, et al. on March 5, 2013 for Medical diagnostic instrument having portable illuminator.

Applicant believes that another reference corresponds to U.S. Patent No. 8,463,364 issued to Wood, et al. on June 11, 2013 for Vein scanner.

Applicant believes that another reference corresponds to U.S. Patent No. 8,838,210 issued to Wood, et al. on September 16, 2014 for Scanned laser vein contrast enhancer using a single laser.

Applicant believes that another reference corresponds to U.S. Patent No. 9,044,207 issued to Goldman, et al. on June 2, 2015 for Micro vein enhancer for use with a vial holder.

Applicant believes that another reference corresponds to U.S. Patent No. 9,061,109 issued to Wood, et al. on June 23, 2015 for Vein scanner with user interface.

Applicant believes that another reference corresponds to U.S. Patent No. 9,186,063 issued to Goldman, et al. on November 17, 2015 for Scanned laser vein contrast enhancer using one laser for a detection mode and a display mode.

Applicant believes that another reference corresponds to U.S. Patent No. 9,492,117 issued to Goldman, et al. on November 15, 2016 for Practitioner-mounted micro vein enhancer.

Applicant believes that another reference corresponds to U.S. Patent Application Publication No. 2012/0101342, published on April 26, 2012 to Duffy, et al. for Pediatric tissue illuminator.

Applicant believes that another reference corresponds to U.S. Patent Application Publication No 2012/0101343, published on April 26, 2012 to Duffy, et al. for Medical imaging device. Applicant believes that another reference corresponds to U.S. Patent Application Publication No. 2015/0094662, published on April 2, 2015 to Lee, et al. for Visualization apparatus for vein. Applicant believes that another reference corresponds to U.S. Patent No. D362,910 issued to Frank C. Creaghan on October 3, 1995 for Instrument for viewing subcutaneous venous structures.

Applicant believes that another reference corresponds to U.S. Patent No. RE33,234 issued to Kim Landry on June 19, 1990 for Transcutaneous intravenous illuminator.

Applicant believes that another reference corresponds to U.S. Patent No. 6,923,762 issued on August 2, 2005 to Creaghan, Jr. for Venoscope apparatus. Other relevant prior art is disclosed in US2011/190638A1.

Other patents describing the closest subject matter provide for a number of more or less complicated features that fail to solve the problem in an efficient and economical way. None of these patents suggest the novel features of the present invention.

### IV. SUMMARY OF THE INVENTION

The invention is defined in claim 1. The present invention is a portable breast light assembly, comprising a housing having a sidewall. The sidewall comprises at least one ventilation hole. An electrical assembly comprises a control panel having a power switch, a timer controller, an intensity controller, a screen, and at least one light source to emit visible light at a predetermined wavelength. The present invention further comprises a cap assembly.

The housing further comprises a top edge and a base. The sidewall further comprises first and second protrusions. The timer controller controls at least one range of operating time. The intensity controller controls at least one level of light intensity. The electrical assembly comprises a sensor. The sensor is connected to circuitry that is connected to the at least one light source. The predetermined wavelength is approximately between 620-780 nm corresponding to a red light region. The electrical assembly comprises a port that receives a plug connector to receive charge from an external source.

The cap assembly comprises a cap edge, a base edge, a cap base and a cap sidewall. The cap base comprises at least one hole. The at least one light source aligns with the at least one hole. The cap sidewall has a first predetermined diameter. The base edge has a second predetermined diameter. The cap sidewall extends from the base edge, and the first predetermined diameter is smaller than the second predetermined diameter.

The cap assembly comprises a cover. The cover is positioned onto the cap base. The cover is secured by the cap edge. The cover contacts the sensor. The cover is transparent to allow the visible light to pass therethrough. The base edge is fixed onto the top edge of the housing. The light emitted by the at least one light source illuminates a breast when the cover is biased against the breast with a predetermined force.

It is therefore one of the main objects of the present invention to provide a portable breast light assembly.

It is another object of this invention to provide a portable breast light assembly designed for light transmission through breast tissue.

It is another object of this invention to provide a portable breast light assembly for breast viewing.

It is another object of this invention to provide a portable breast light assembly that is volumetrically efficient for carrying, transporting, and storage.

It is another object of this invention to provide a portable breast light assembly, which is of a durable and reliable construction.

It is yet another object of this invention to provide a portable breast light assembly that is inexpensive to maintain while retaining its effectiveness.

Further objects of the invention will be brought out in the following part of the specification, wherein detailed description is for the purpose of fully disclosing the invention without placing limitations thereon.

### V. BRIEF DESCRIPTION OF THE DRAWINGS

With the above and other related objects in view, the invention consists in the details of construction and combination of parts as will be more fully understood from the following description, when read in conjunction with the accompanying drawings in which:
**Figure 1** is an isometric view of the present invention.
**Figure 2** is a front view of the present invention with a cut view at the cap assembly and housing.
**Figure 3** is a rear view of the present invention.
**Figure 4** is a view of the present invention in use illuminating a breast.

### VI. DETAILED DESCRIPTION OF THE PREFERRED EMBODIMENT

Referring now to the drawings, the present invention is a portable breast light assembly and is generally referred to with numeral **10.** It can be observed that it basically includes housing **20,** electrical assembly **40,** and cap assembly **60.**

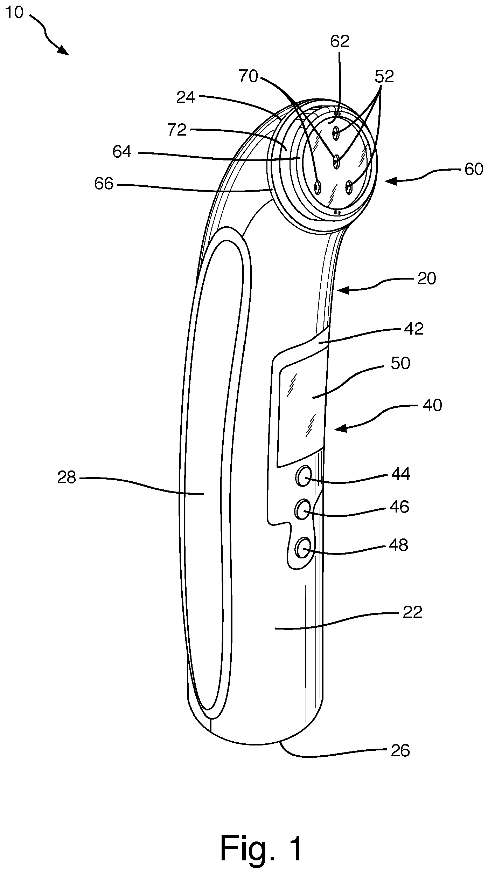

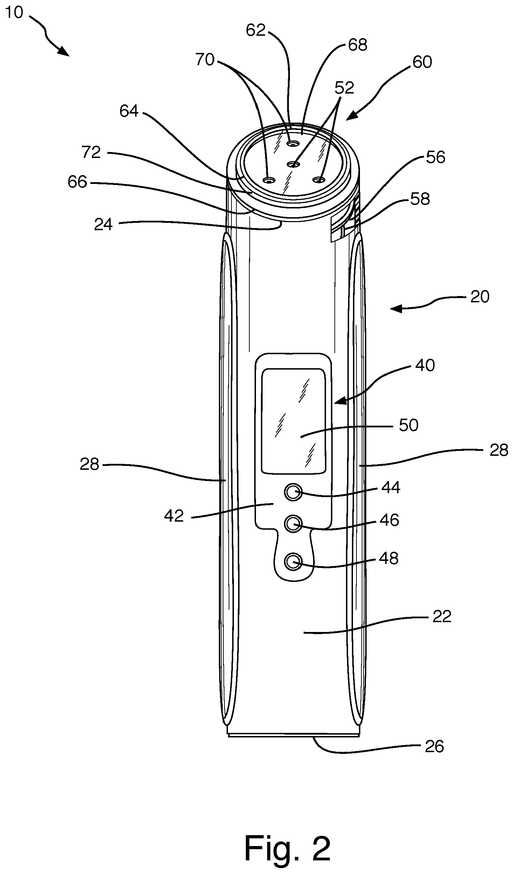

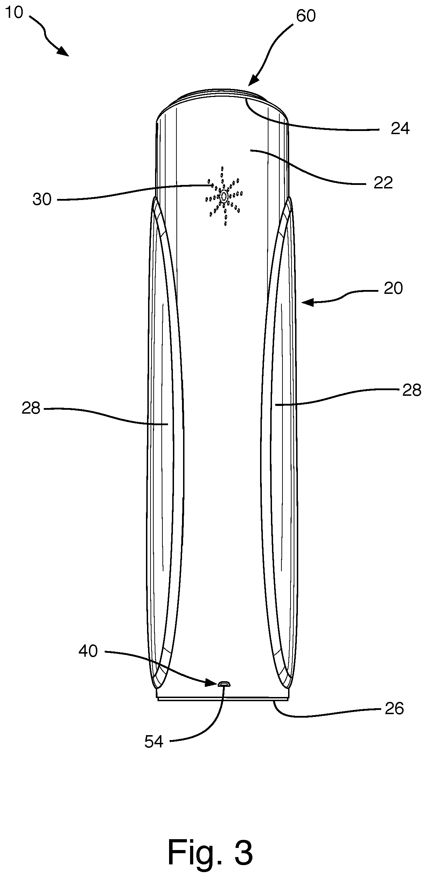

As seen in figures 1 and 2, housing **20** has sidewall **22** that comprises top edge **24** and base **26.** Sidewall **22** further comprises at least one ventilation hole **30,** seen in figure 3.

As also seen in figures 1 and 2, electrical assembly **40** comprises control panel **42** having power switch **44,** timer controller **46,** intensity controller **48,** screen **50,** at least one light source **52,** and a rechargeable battery, not seen. Power switch **44** is an on / off switch. Timer controller **46** controls at least one range of operating time. In a preferred embodiment, there are two different ranges of operating time. As an example the operating times may be 2 or 5 minutes. It is noted however that the operating times may be lesser or greater. Intensity controller **48** controls at least one level of light intensity. In a preferred embodiment, there are four different intensity levels. The light intensity level is incremented or reduced to optimally illuminate a breast when cover **62** is biased against a breast with a predetermined force. The light intensity level may depend on factors such as, but not limited to, the size and firmness of the breast.

In a preferred embodiment, screen **50** is a liquid-crystal display that turns on when power switch **44** is in the "on" position. Screen **50** shows a status of parameters including operating time, intensity level, and battery charge.

At least one light source **52** emits visible light at a predetermined wavelength. In a preferred embodiment, the predetermined wavelength is approximately between 620-780 nm corresponding to a red light region. At least one light source **52** may be a plurality of light-emitting diodes (LED).

Cap assembly **60** comprises cap edge **64,** base edge **66,** cap base **68,** and cap sidewall **72.** Base edge **66** is fixed to top edge **24** of housing **20.** Cap base **68** comprises at least one hole **70.** Each of at least one light source **52** aligns with holes **70.** In a preferred embodiment, there are four holes **70** with respective light sources **52.** In addition, cap sidewall **72** has a first predetermined diameter. Base edge **66** has a second predetermined diameter. Cap sidewall **72** extends from base edge **66.** The first predetermined diameter is smaller than the second predetermined diameter. Cover **62** is positioned onto cap base **68** and is secured by cap edge **64.** Cover **62** is transparent to allow the visible light to pass therethrough.

As seen in figure 2, housing **20** further comprises first and second protrusions **28** for easier handling and/or gripping. Control panel **42** is positioned between first and second protrusions **28.**

Electrical assembly **40** further comprises sensor **56.** Sensor **56** is connected to circuitry **58** that is connected to at least one light source **52.**

Circuitry **58** is a touch detection circuitry that is connected with a single chip computer, which has a built-in debounce circuitry. Circuitry **58** comprises a touch input contact connected to sensor **56** and a touch output contact connected to at least one light source **52.** Cover **62** contacts sensor **56** by pressure or a predetermined force.

When cover **62** touches the skin, such as when cover **62** is biased against a breast with a predetermined force, the touch output contact outputs a higher intensity of light.

When cover **62** does not touch the skin, such as when cover **62** is not biased against a breast, the touch output contact outputs a lower intensity of light. Therefore the light intensity changes from lower to higher by skin contact due to sensor **56.**

As seen in figure 3, sidewall **22** has at least one ventilation hole **30.** Due to electrical assembly **40,** and particularly light sources **52** seen in figure 1 and 2, portable breast light assembly **10** may become warm during usage. Ventilation holes **30** are designed to counteract this effect. In a preferred embodiment, there are a predetermined number of ventilation holes **30** near cap assembly **60.** Electrical assembly **40** further comprises port **54** that receives a plug connector, not seen, to receive charge from an external source.

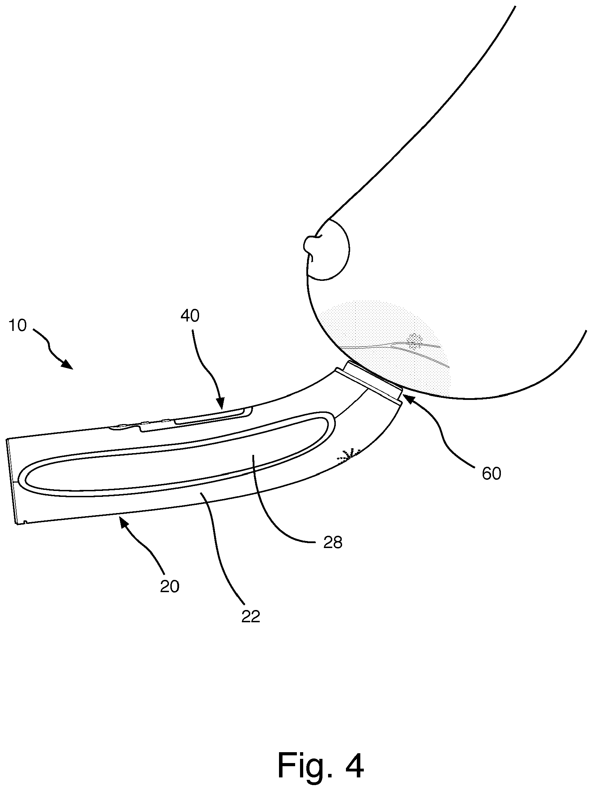

As seen in figure 4, light emitted by at least one light source **52,** seen in figures 1 and 2, illuminates a breast when cover **62** is biased against the breast with a predetermined force. Portable breast light assembly **10** is therefore designed to maximize light transmission through breast tissue. Therefore, present invention **10** works by shining a powerful red light through the breast tissue, whereby the predetermined wavelength of the red light is approximately between 620-780 nm corresponding to a red light region. As a result, one can see veins and blood vessels. Around the nipple, one may also see small dots, which are part of the mammary glands.

For optimal use of present invention **10:**
A) charge portable breast light assembly **10** before use;
B) make a room as dark as possible. The darker the room, the easier to use portable breast light assembly **10.** Wait a few minutes to allow the eyes to get used to the dark. Turn on portable breast light assembly **10;**
C) for each breast: lubricate a breast to allow cover **62** to slide across the breast more easily. Apply the lubricant liberally over the entire breast. In a preferred embodiment, utilize water-based lubricants;
D) in front of a mirror while standing or sitting, hold and keep portable breast light assembly **10** against the breast with a predetermined force. The breast itself will suddenly appear brighter. Dark lines are veins and other blood vessels are in silhouette; and
E) slide portable breast light assembly **10** around the breast while keeping the portable breast light assembly **10** against the breast with the predetermined force.

The foregoing description conveys the best understanding of the objectives and advantages of the present invention. Different embodiments may be made of the inventive concept of this invention. It is to be understood that all matter disclosed herein is to be interpreted merely as illustrative, and not in a limiting sense.

### VII. INDUSTRIAL APPLICABILITY

It is evident that an invention such as the portable breast light assembly claimed in the present application is quite desirable because it is designed for light transmission through breast tissue for breast viewing. Also, the present invention is quite desirable because it is volumetrically efficient for carrying, transporting, and storage; is of a durable and reliable construction; and it is inexpensive to maintain while retaining its effectiveness.

## Claims

1. Portable breast light assembly (10), consisting of:
**A)** a housing (20) having a sidewall, said sidewall has at least one ventilation hole and first and second protrusions, said housing further has a top edge and a base;
**B)** an electrical assembly (40) having a control panel that is positioned between said first and second protrusions that are for handling and gripping, said control panel also having a power switch that is an on / off switch, a timer controller, an intensity controller, a screen, and at least one light source that is a plurality of light-emitting diodes to emit visible light at a predetermined wavelength, said screen is a liquid-crystal display that turns on when said power switch is in an on position, said timer controller controls at least one range of operating time, and said screen shows a status of said operating time, said intensity controller controls at least one level of light intensity, and said screen shows a status of said light intensity and battery charge, said electrical assembly further has a sensor, said sensor is connected to circuitry that is connected to said at least one light source, said circuitry is a touch detection circuitry that is connected with a single chip computer, which has a built-in debounce circuitry, said circuitry has a touch input contact connected to said sensor and a touch output contact connected to said at least one light source, said touch output contact outputs at least two light intensities, wherein one is larger than the other, whereby said predetermined wavelength is between 620-780 nm corresponding to a red light region, whereby said electrical assembly further has a port that receives a plug connector to receive charge from an external source; and
**C)** a cap assembly (60) having a cap edge, a base edge, a cap base and a cap sidewall, said cap base has at least one hole, at least one light source aligns with said at least one hole, said cap sidewall has a first predetermined diameter, said base edge has a second predetermined diameter, said cap sidewall extends from said base edge, and said first predetermined diameter is smaller than said second predetermined diameter, cap assembly further has a cover that is positioned onto said cap base and is secured by said cap edge, said cover contacts said sensor by pressure or a predetermined force, and said cover is transparent to allow said visible light to pass therethrough, and said base edge is fixed onto said top edge of said housing, said at least one hole are four holes that aligns with four of said at least one light source respectively, said screen is a first predetermined distance from said cap assembly, said power switch is a second predetermined distance from said cap assembly, and said second predetermined distance is greater than said first predetermined distance, and said timer controller is a third predetermined distance from said cap assembly, and said third predetermined distance is greater than said second predetermined distance, and said intensity controller is a fourth predetermined distance from said cap assembly, and said fourth predetermined distance is greater than said third predetermined distance;
wherein said portable breast light assembly is further configured to increment or reduce said at least one level of light intensity to illuminate a breast, whereby said visible light emitted by said at least one light source illuminates said breast when said cover is biased against said breast with said predetermined force, whereby said touch output contact outputs a higher intensity of said visible light to enable light transmission through breast tissue to see veins, blood vessels, and part of mammary glands, and when said cover is not biased against said breast, said touch output contact outputs a lower intensity of said visible light, and said light intensity level depends on a size and firmness of said breast, said veins and said blood vessels appear in silhouette when said cover is biased against said breast with said predetermined force, and bias said cover against said breast with said predetermined force, after application of a water-based lubricant to said breast.

## Patentansprüche

1. Tragbare Brustleuchtenanordnung (10), bestehend aus:
**A)** einem Gehäuse (20) mit einer Seitenwand, wobei die Seitenwand mindestens ein Belüftungsloch und erste und zweite Vorsprünge aufweist, wobei das Gehäuse außerdem eine Oberkante und eine Basis aufweist;
**B)** einer elektrischen Baugruppe (40) mit einem Bedienfeld, das zwischen den ersten und zweiten Vorsprüngen positioniert ist, die der Handhabung und dem Greifen dienen, wobei das Bedienfeld außerdem einen Netzschalter, der ein Ein-/Ausschalter ist, eine Zeitsteuerung, eine Intensitätssteuerung, einen Bildschirm und mindestens eine Lichtquelle aufweist, die aus mehreren Leuchtdioden besteht, um sichtbares Licht mit einer vorgegebenen Wellenlänge zu emittieren, wobei der Bildschirm eine Flüssigkristallanzeige ist, die sich einschaltet, wenn der Netzschalter eingeschaltet ist, die Zeitsteuerung mindestens einen Bereich der Betriebszeit steuert und der Bildschirm einen Status der Betriebszeit anzeigt, die Intensitätssteuerung mindestens einen Grad der Lichtintensität und der Bildschirm einen Status der Lichtintensität und der Batterieladung anzeigt, wobei die elektrische Baugruppe ferner einen Sensor aufweist, wobei dieser Sensor mit einer Schaltung verbunden ist, die mit der mindestens einen Lichtquelle verbunden ist, wobei die Schaltung eine Berührungserkennungsschaltung ist, die mit einem Ein-Chip-Computer verbunden ist, der über eine integrierte Entprellungsschaltung verfügt, die Schaltung einen Berührungseingabekontakt aufweist, der mit dem Sensor verbunden ist, und einen Berührungsausgabekontakt, der mit der mindestens einen Lichtquelle verbunden ist, wobei der Berührungsausgangskontakt mindestens zwei Lichtintensitäten ausgibt, wobei eine größer als die andere ist, wobei die vorgegebene Wellenlänge zwischen 620-780 nm liegt, was einem roten Lichtbereich entspricht, wobei die elektrische Baugruppe außerdem über einen Anschluss verfügt, der einen Stecker aufnimmt, um Ladung von einer externen Quelle zu empfangen; und
**C)** einer Kappenanordnung (60) mit einer Kappenkante, einer Basiskante, einer Kappenbasis und einer Kappenseitenwand, wobei die Kappenbasis mindestens ein Loch aufweist, mindestens eine Lichtquelle auf das mindestens eine Loch ausgerichtet ist, die Kappenseitenwand einen ersten vorgegebenen Durchmesser aufweist, die Basiskante einen zweiten vorgegebenen Durchmesser aufweist, die Kappenseitenwand sich von der Basiskante erstreckt und der erste vorgegebene Durchmesser kleiner als der zweite vorgegebene Durchmesser ist, die Kappenanordnung ferner eine Abdeckung aufweist, die auf der Kappenbasis positioniert ist und durch den Kappenrand fixiert wird, die Abdeckung den Sensor durch Druck oder eine vorbestimmte Kraft berührt, und die Abdeckung transparent ist, um den Durchtritt des sichtbaren Lichts zu ermöglichen, und der Basisrand an der Oberkante des Gehäuses wobei das mindestens eine Loch vier Löcher sind, die jeweils mit vier der mindestens einen Lichtquelle ausgerichtet sind, der Bildschirm einen ersten vorbestimmten Abstand von der Kappenanordnung aufweist, der Netzschalter einen zweiten vorbestimmten Abstand von der Kappenanordnung hat und der zweite vorbestimmte Abstand größer ist als der erste vorgegebene Abstand und die Zeitsteuerung einen dritten vorgegebenen Abstand von der Kappenanordnung aufweist und der dritte vorgegebene Abstand größer als der zweite vorgegebene Abstand ist und die Intensitätssteuerung einen vierten vorgegebenen Abstand von der Kappenanordnung aufweist und der vierte vorgegebene Abstand größer als der dritte vorgegebene Abstand ist;
wobei die tragbare Brustleuchtenanordnung außerdem zum Erhöhen oder Verringern des mindestens einen Lichtintensitätsgrads konfiguriert ist, um eine Brust zu beleuchten, wobei das von der mindestens einen Lichtquelle emittierte sichtbare Licht die Brust beleuchtet, wenn die Abdeckung mit der vorbestimmten Kraft gegen die Brust gedrückt wird, wodurch der Berührungsausgabekontakt eine höhere Intensität des sichtbaren Lichts ausgibt, um die Lichtübertragung durch das Brustgewebe zu ermöglichen, um Venen, Blutgefäße und Teile der Brustdrüsen zu sehen, und wenn die Abdeckung nicht gegen die Brust gedrückt wird, der Berührungsausgabekontakt eine geringere Intensität ausgibt, wobei das sichtbare Licht und der Lichtintensitätsgrad von der Größe und Festigkeit der Brust abhängen, die Venen und Blutgefäße als Silhouette erscheinen, wenn die Abdeckung mit der vorgegebenen Kraft gegen die Brust gedrückt wird, und wenn die Abdeckung mit der vorgegebenen Kraft nach Auftragen eines Gleitmittels auf Wasserbasis auf die Brust gegen die Brust gedrückt wird.

## Revendications

1. Ensemble lumière mammaire portable (10), composé de :
**A)** un boîtier (20) comportant une paroi latérale, ladite paroi latérale comportant au moins un trou de ventilation et des première et seconde saillies, ledit boîtier comportant en outre un bord supérieur et une base ;
**B)** un ensemble électrique (40) comportant un panneau de commande qui est positionné entre lesdites première et seconde saillies destinées à la manipulation et à la préhension, ledit panneau de commande comportant également un interrupteur d'alimentation qui est un interrupteur marche/arrêt, un organe de commande de minuterie, un variateur d'intensité, un écran, et au moins une source de lumière qui est une pluralité de diodes électroluminescentes pour émettre une lumière visible à une longueur d'onde prédéterminée, ledit écran est un affichage à cristaux liquides qui s'allume lorsque ledit interrupteur d'alimentation est en position marche, ledit organe de commande de minuterie commande au moins une plage de temps de fonctionnement, et ledit écran affiche un état dudit temps de fonctionnement, ledit variateur d'intensité règle au moins un niveau d'intensité lumineuse, et ledit écran affiche un état de ladite intensité lumineuse et de la charge de la batterie, ledit ensemble électrique comporte en outre un capteur, ledit capteur est connecté aux circuits qui sont connectés à ladite au moins une source de lumière, lesdits circuits sont des circuits de détection tactile qui sont connectés à un ordinateur à puce unique, qui possède des circuits anti-rebond intégrés, lesdits circuits ont un contact d'entrée tactile connecté audit capteur et un contact de sortie tactile connecté à ladite au moins une source de lumière, ledit contact de sortie tactile émet au moins deux intensités lumineuses, l'une étant plus grande que l'autre, ladite longueur d'onde prédéterminée étant de ce fait comprise entre 620-780 nm correspondant à une région de lumière rouge, ledit ensemble électrique comportant de ce fait en outre un port qui reçoit un connecteur enfichable pour recevoir une charge provenant d'une source externe ; et
**C)** un ensemble capuchon (60) comportant un bord de capuchon, un bord de base, une base de capuchon et une paroi latérale de capuchon, ladite base de capuchon comporte au moins un trou, au moins une source de lumière s'aligne avec ledit au moins un trou, ladite paroi latérale de capuchon présente un premier diamètre prédéterminé, ledit bord de base présente un second diamètre prédéterminé, ladite paroi latérale de capuchon s'étend à partir dudit bord de base, et ledit premier diamètre prédéterminé est inférieur audit second diamètre prédéterminé, l'ensemble capuchon comporte en outre un couvercle qui est positionné sur ladite base de capuchon et est fixé par ledit bord de capuchon, ledit couvercle entre en contact avec ledit capteur par pression ou par une force prédéterminée, et ledit couvercle est transparent pour permettre à ladite lumière visible de passer à travers celui-ci, et ledit bord de base est fixé sur ledit bord supérieur dudit boîtier, ledit au moins un trou est quatre trous qui s'alignent respectivement avec quatre de ladite au moins une source de lumière, ledit écran est à une première distance prédéterminée dudit ensemble capuchon, ledit interrupteur d'alimentation est à une troisième distance prédéterminée dudit ensemble capuchon, et ladite deuxième distance prédéterminée est supérieure à ladite première distance prédéterminée, et ledit organe de commande de minuterie est à une troisième distance prédéterminée dudit ensemble capuchon, et ladite troisième distance prédéterminée est supérieure à ladite deuxième distance prédéterminée, et ledit variateur d'intensité est à une quatrième distance prédéterminée dudit ensemble capuchon, et ladite quatrième distance prédéterminée est supérieure à ladite troisième distance prédéterminée ;
dans lequel ledit ensemble lumière mammaire portable est en outre conçu pour augmenter ou pour réduire ledit au moins un niveau d'intensité lumineuse pour éclairer un sein, ladite lumière visible émise par ladite au moins une source de lumière illuminant de ce fait ledit sein lorsque ledit couvercle est sollicité contre ledit sein avec ladite force prédéterminée, ledit contact de sortie tactile produit de ce fait une intensité plus élevée de ladite lumière visible pour permettre la transmission de la lumière à travers le tissu mammaire pour voir les veines, les vaisseaux sanguins et une partie des glandes mammaires, et quand ledit couvercle n'est pas sollicité contre ledit sein, ledit contact de sortie tactile produit une intensité plus faible de ladite lumière visible, et ledit niveau d'intensité lumineuse dépend de la taille et de la fermeté dudit sein, lesdites veines et lesdits vaisseaux sanguins apparaissent en silhouette lorsque ledit couvercle est sollicité contre ledit sein avec ladite force prédéterminée, et sollicite ledit couvercle contre ledit sein avec ladite force prédéterminée, après application d'un lubrifiant à base d'eau sur ledit sein.
